(19) Europäisches Patentamt
European Patent Office
Office européen des brevets

(11) EP 0 751 125 A1

# (12) EUROPÄISCHE PATENTANMELDUNG

(43) Veröffentlichungstag:
**02.01.1997 Patentblatt 1997/01**

(21) Anmeldenummer: **96109672.4**

(22) Anmeldetag: **17.06.1996**

(51) Int. Cl.[6]: **C07C 403/20**, C07C 403/18, C07D 311/72, C07C 401/00, C07H 13/04, A61K 31/20, A61K 31/07, A61K 31/595, A61K 31/355, A61K 31/70

(84) Benannte Vertragsstaaten:
**CH DE FR GR LI NL**

(30) Priorität: **26.06.1995 DE 19523079**

(71) Anmelder: **BASF AKTIENGESELLSCHAFT
67056 Ludwigshafen (DE)**

(72) Erfinder:
- **John, Michael, Dr.
67245 Lambsheim (DE)**
- **Siegel, Wolfgang, Dr.
67117 Limburgerhof (DE)**

## (54) Ester und Amide der 9 (Z)-Retinsäure

(57) Ester und Amide der 9(Z)-Retinsäure zur Behandlung von Carcinomen, neue 9(Z)-Retinsäurederivate, Pharmazeutische Zusammensetzungen enthaltend als antikrebswirksame Wirkstoffe Ester und Amide der 9(Z)-Retinsäure sowie die Verwendung von Estern und Amiden der 9(Z)-Retinsäure zur Behandlung und Prophylaxe von Carcinomen.

EP 0 751 125 A1

Printed by Rank Xerox (UK) Business Services
2.13.11/3.4

## Beschreibung

Die Erfindung betrifft Ester und Amide der 9(Z)-Retinsäure zur Behandlung von Carcinomen, neue 9(Z)-Retinsäurederivate sowie pharmazeutische Zusammensetzungen enthaltend Ester und Amide der 9(Z)-Retinsäure sowie die Verwendung derselben zur Behandlung von Carcinomen

Retinsäuren (Vitamin-A-Säuren) sind Derivate von Vitamin-A (Retinol), die ein großes Spektrum biologischer Aktivitäten, wie celluläre Differenzierung u.v.a. umfassen. Ihre biologische Wirksamkeit wird durch die Wechselwirkung mit zwei Familien von Kernrezeptoren (RA-Rezeptor und RX-Rezeptor) ermöglicht (vgl. Mol. Cell. Biol. 14 Nr.4 (1994) Seiten 2323 - 30).

Die Anwendung von all-(E)- oder 13-(Z)- Retinsäuren oder deren Derivaten in der Therapie von Akne ist lange bekannt. (vgl. US 3,729,568 und EP 366 713). Eine Verwendung von 9-(Z)-Retinsäure in der Krebsbehandlung wird erst seit der Identifizierung spezifischer Rezeptorsysteme erwogen [vgl. Cell 68 (1992) S. 397 - 406; Nature 361 (1993) S. 657 - 60; Nucl. Acid Research 21, Nr. 5 (1993) S. 1231 - 37; Differentiation 54 (1993) S. 123 - 29; J. Bio. Chem. 269 (1994) S. 16689 - 95, Blood 82, Nr. 12 (1993) S. 3592 - 99; Blood 82, Nr. 10 (1993) S.22].

Wie bereits von den Strukturisomeren all-E- und 13 (Z)-Retinsäure bekannt ist, treten auch bei der Anwendung von 9(Z)-Retinsäure teilweise starke Nebenwirkungen auf, so daß das Ausmaß an Nebenreaktionen die erzielte Wirkung überproportional überkompensiert. Zudem führt die topische Anwendung der freien 9(Z)-Retinsäure häufig zu starken Hautreizungen und Schmerzen für den Patienten in Abhängigkeit von Konzentration und Häufigkeit der Applikation. Art und Zahl der Nebenwirkungen reduziert die Möglichkeit einer Therapie mit hohen Dosen an freier 9(Z)-Retinsäure und schränkt die Anzahl der Applikationsgebiete ein.

Es wurde nun gefunden, daß bestimmte Ester und Amide der 9(Z)-Retinsäure auch zur Behandlung von Carcinomen verwendet werden können, dabei aber nur wenige, wenn überhaupt, Nebenwirkungen zeigen. Die vorliegende Erfindung betrifft daher leicht herstellbare Derivate von 9(Z)-Retinsäure, die von Bedeutung für die Behandlung von Carcinomen unter gleichzeitiger Minimierung von toxikologischen Nebenwirkungen der freien 9(Z)-Vitamin-A-Säure sind.

Gegenstand der Erfindung sind daher Verbindungen für die Verwendung zur Behandlung von Carcinomen der allgemeinen Formel I

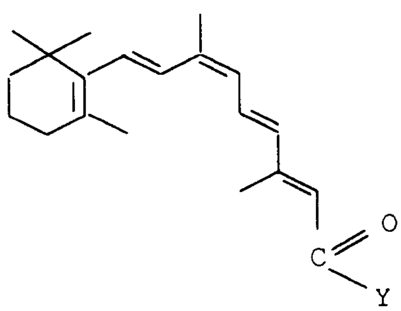

(I) ,

in der Y für folgende Reste stehen kann:

$$-O-CR^1_2-CO-R^2,$$

worin $R^1$

für H oder $C_1$-bis $C_{20}$-Alkyl oder $C_1$-bis $C_{20}$-Alkanoyl, vorzugsweise für H oder $C_1$- bis $C_4$-Alkyl steht und

$R^2$ für $C_1$-bis $C_4$-Alkyl, $-CH_2OH$, $-NR^3_2$ , $-CH_2-O-CO-R^3$,

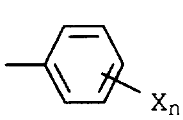 , 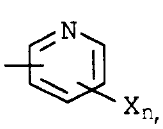 , 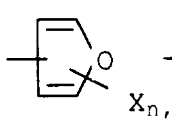 , 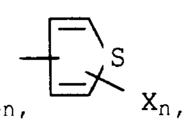 , oder 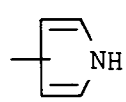

steht, und worin $R^3$ für H oder $C_1$-bis $C_4$-Alkyl steht,

X für H, F, Cl, Br, J, -OH, $-OR^3$, $-NH_2$, $-NO_2$, -CN, $-NHR^3$, $-NR^3_2$ , -SH, $-SR^3$, $-O-CO-R^3$, $-CO-R^3$, -CHO oder $-CO-OR^3$

und n für eine ganze Zahl von 0 bis 5 steht

Oder aber Y für

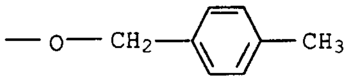 ; 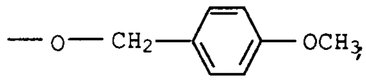

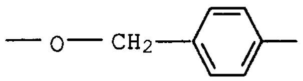 ; 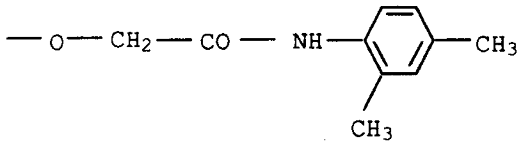 ;

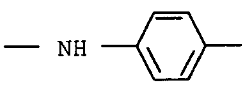 ; 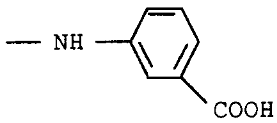 ;

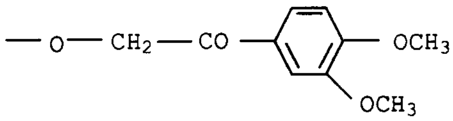

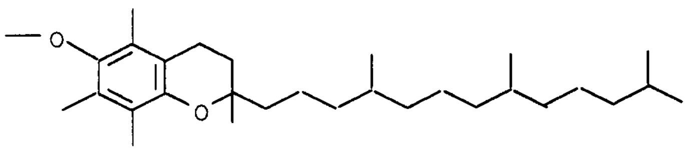 ;

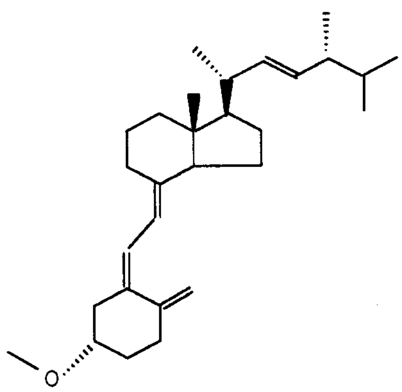

$-O-CHR^4_2$ ; $-O-CHR^3-O-CO-OR^3$

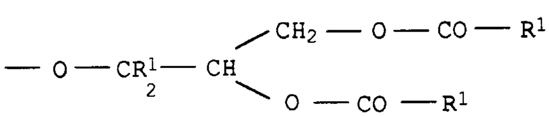

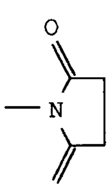 ; 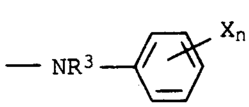 ; 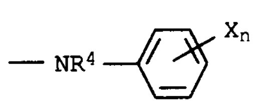 ,

worin $R^1$, $R^3$, X und n die oben angegebene Bedeutung haben und $R^4$ für $-CO-OR^3$, $-CO-R^3$ oder

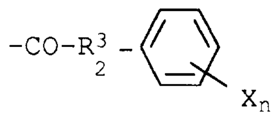

steht,

wobei in den Verbindungen, in denen mehrere Reste $R^1$, $R^2$, $R^3$ und/oder $R^4$ enthalten sind, diese gleich oder verschieden sein können, oder aber

Y für einen der Reste

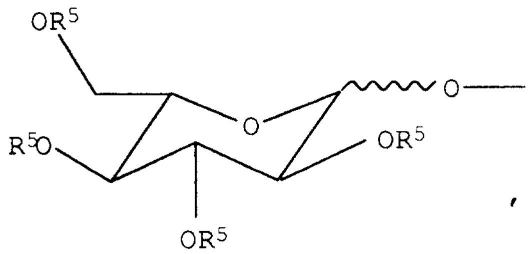

,

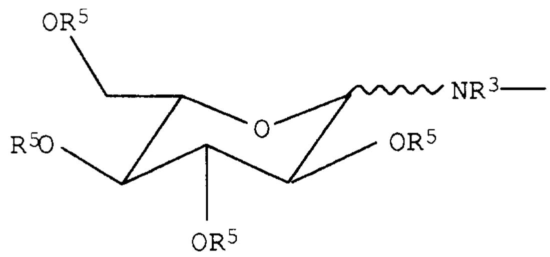

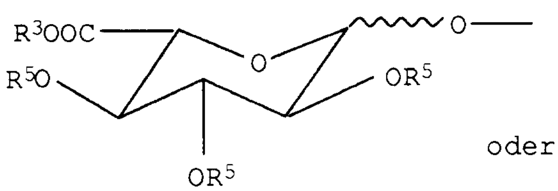

oder

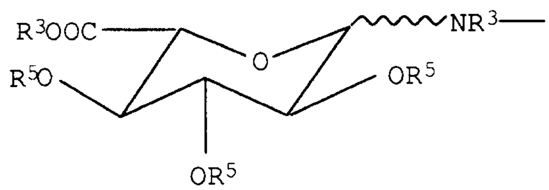

steht,

in der $R^3$ die oben angegebene Bedeutung hat und $R^5$ für H oder einen $C_1$-bis $C_4$-Alkylrest oder $C_1$ -bis $C_4$-Alkanoylrest steht.

Von besonderer Bedeutung für die Verwendung bei der Behandlung von Carcinomen sind Verbindungen der allgemeinen Formel I worin Y für den Rest

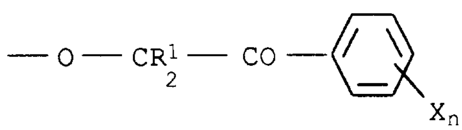

steht, in der $R^1$ für H,

X für F, -OH, $-OCH_3$, $-NH_2$, -CN- $-NHCH_3$ oder $-N(CH_3)_2$ steht und n eine ganze Zahl von 1 bis 5, vorzugsweise 1 bis 3, insbesondere 1 bis 2, bedeutet,

sowie die Verbindungen der allgemeinen Formel I,

worin Y für einen der Reste

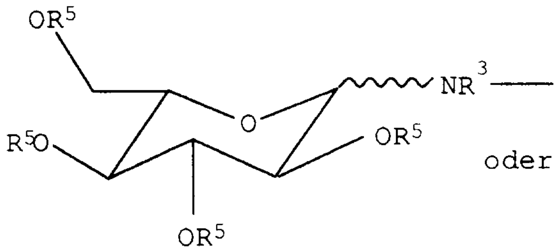

oder

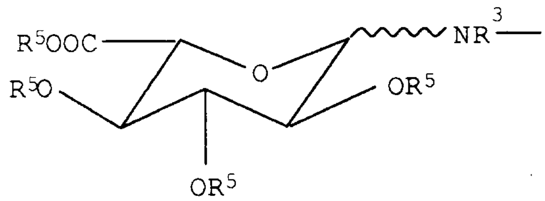

steht,
worin $R^5$ H oder $C_1$-bis $C_4$-Alkanoyl, wie $CH_3$-CO-, $C_2H_5$-CO-, $C_3H_7$-CO oder aber HCO- und $R^3$ H, $C_1$-bis $C_4$-Alkyl oder $C_1$-bis $C_4$-Alkanoyl bedeuten,
insbesondere
Verbindungen der allgemeinen Formel I, in der Y einen der Reste

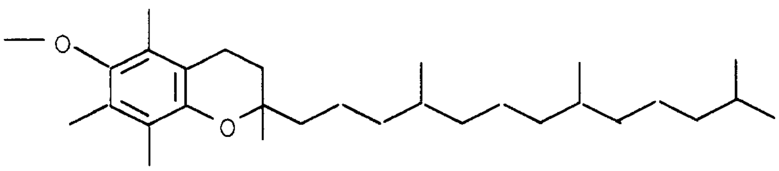

;

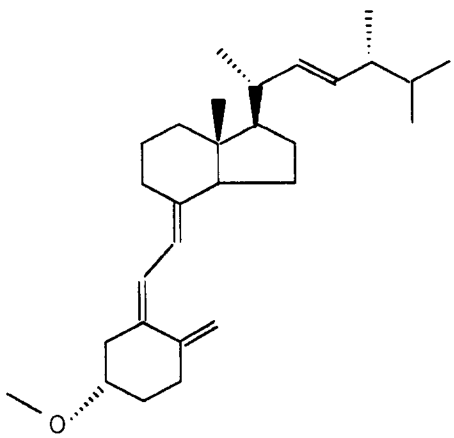

;

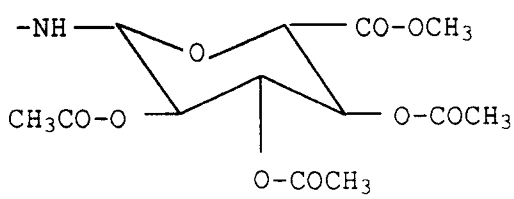

oder

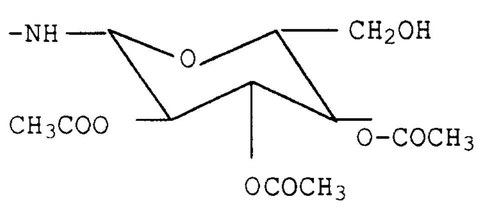

bedeuten.

Die erfindungsgemäßen 9(Z)-Retinsäurederivate können sowohl lokal, d.h. topisch zur Behandlung von Hautkrebs als auch oral, d.h. systemisch, zur Behandlung von Carcinomen oder von Praecancerosen verwendet werden

Unter bestimmten Voraussetzungen ist aber auch eine lokale und systemische Prophylaxe von Carcinomen möglich.

Gegenstand der Erfindung sind daher auch pharmazeutische Zusammensetzungen enthaltend Verbindungen der allgemeinen Formel I neben üblichen pharmazeutischen Trägerstoffen oder Verdünnungsmitteln und üblichen pharmazeutisch-technischen Hilfsstoffen sowie die Verwendung von Verbindungen der allgemeinen Formel I in Form von Pasten, Gelen, Salben, Cremes, Lotionen, Puder, Lösungen oder Emulsionen enthaltend 0,001 bis 5 % dieser Verbindungen für die lokale Behandlung von Carcinomen oder Praecancerosen sowie die Verwendung einer Verbindung der allgemeinen Formel I in Form von Tabletten, Filmtabletten, Dragees, Kapseln, Pillen, Pulver, Granulat, Lösungen oder Suspensionen für die systemische Behandlung von Carcinomen oder Praecancerosen und die Verwendung von pharmazeutischen Zusammensetzungen enthaltend Verbindungen der allgemeinen Formel I für die lokale oder systemische Prophylaxe von Carcinomen.

Die Herstellung der therapeutischen Mittel oder Zubereitungen mit üblichen Trägerstoffen oder Verdünnungsmitteln und gegebenenfalls üblichen pharmazeutisch-technischen Hilfsstoffen, entsprechend der gewünschten Applikationsart mit einer zur Anwendung geeigneten Dosierung erfolgt in an sich bekannter Weise, insbesondere durch Vermischen. Die therapeutischen Mittel enthalten die erfindungsgemäß zu verwendenden Verbindungen bei lokaler Anwendung in Mengen von 0,001 bis 5 Gew.-%, bevorzugt in 0,001 bis 1 Gew.-% und bei systemischer Anwendung vorzugsweise in einer Einzeldosis von 0,1 bis 10 mg.

Tests bezüglich der topischen Anwendung werden durch allgemein übliche Testprogramme in der Onkologie ausgeführt.

Getestet wird die Unterdrückung von unkontrolliertem Zellwachstum.

Die Herstellung von erfindungsgemäßen Verbindungen der allgemeinen Formel I wird durch die folgenden Beispiele aufgezeigt.

Beispiel 1

2-[9(Z)-Retinoyloxy]-4'-methyl-acetophenon

50 ml Dimethylformamid wurden im Kolben vorgelegt. Anschließend wurden 0,5 g Kaliumcarbonat und 1,12 g 2-Chlor(4'-methyl)acetophenon sowie 2 g 9(Z)-Retinsäure unter Lichtausschluß zugegeben. Unter Stickstoff wurde der Ansatz für 24 Stunden (h) bei Raumtemperatur (RT) gerührt. Anschließend wurde das Reaktionsgemisch mit 100 ml Wasser versetzt und für 2 h nachgerührt. Die resultierenden Kristalle wurden abgesaugt und 12 h bei Temperaturen von 30°C unter Stickstoff getrocknet. Man erhielt 2,4 g (entsprechend einer Ausbeute von 84 % der Theorie) des oben genannten Produkts. Die Reinigung des Wertprodukts erfolgte durch Umkristallisation aus Isopropanol. Der Schmelzpunkt Fp des umkristallisierten Produkts betrug 126°C. Die NMR-Spektren stimmen mit der formulierten Struktur überein.

Beispiel 2

2-[9(Z)-Retinoyloxy]-4'-methoxy-acetophenon

Ein Gemisch aus 35 ml Dimethylformamid, 0,5 g Kaliumcarbonat, 1,23 g 2-Chlor-4'-methoxy-acetophenon und 2 g 9(Z)-Retinsäure wurde unter $N_2$ und unter Lichtausschluß bei RT für 24 h gerührt. Anschließend wurde das Reaktionsgemisch bei 30°C mit 100 ml Wasser versetzt und 2 h nachgerührt. Die resultierenden Kristalle wurden abgesaugt und 12 h unter Stickstoff getrocknet. Man erhielt 2,5 g (entsprechend 88,1 % d. Th.) des oben genannten Produktes. Nach Umkristallisieren aus Isopropanol betrug der Schmelzpunkt 124-126°C.

Beispiel 3

2-[9(Z)-Retinoyloxy]-4'-fluor-acetophenon

Ein Gemisch aus 30 ml Dimethylformamid, 0,2 g Kaliumcarbonat, 0,54 g 2-Chlor-4'-fluor-acetophenon und 1 g 9(Z)-Retinsäure wurde unter $N_2$ und Lichtausschluß bei RT 24 h gerührt. Anschließend wurde mit 100 ml Wasser versetzt und 2 h nachgerührt. Nach Abtrennung der Kristalle und 12stündiger Trocknung unter Stickstoff wurden 1,1 g (84,6 % d. Th) des oben genannten Produktes erhalten. Nach Reinigung durch Umkristallisation aus Methanol betrug der Schmelzpunkt 99°C.

Beispiel 4

2-[9(Z)-Retinoyloxy]-3'-fluor-acetophenon

Ein Gemisch aus 50 ml Dimethylformamid, 0,75 g Kaliumcarbonat, 1,8 g 2-Chlor-3'-fluor-acetophenon und 3 g 9(Z)-Retinsäure wurde unter Stickstoff und Lichtausschluß bei RT 24 h gerührt. Anschließend wurde das Reaktionsgemisch mit 100 ml Wasser versetzt und 2 h nachgerührt. Die resultierenden Kristalle wurden abgesaugt und aus Isopropanol umkristallisiert. Es wurden 3,3 g (85 % d. Th.) des oben genannten Produkts mit einem Schmelzpunkt von 76-79°C erhalten.

Beispiel 5

2-[9(Z)-Retinoyloxy]-N-(2',6'-dimethyl-phenyl)-acetamid

Ein Gemisch aus 35 ml Dimethylformamid, 0,5 g Kaliumcarbonat, 1,3 g 2-Chlor-N-(2',6'-dimethyl-phenyl)-acetamid und 2 g 9 (Z)-Retinsäure ließ man unter Stickstoff und Lichtausschluß für 24 h bei RT rühren. Anschließend versetzte man das Reaktionsgemisch mit 100 ml Wasser und rührte dann 2 h nach. Die erhaltenen Kristalle wurden 12 h lang unter Stickstoff getrocknet. Durch Umkristallisation aus Isopropanol wurden 2,4 g (91 % d. Th.) des oben genannten Produkts mit einem Festpunkt Fp= 147-148°C erhalten.

Beispiel 6

9(Z)-Retinsäure-N-(p-carboxy-phenyl)-amid

Ein Gemisch aus 10 g 9(Z)-Retinsäure, 2,9 g Pyridin in 650 ml Diethylether wurde auf 0°C abgekühlt. Anschließend wurden bei 0°C 3,5 g Thionylchlorid zugetropft und 2 h bei 0°C nachgerührt. Ausfallendes Pyridiniumhydrochlorid wurde abgetrennt. Das Filtrat wurde danach bei -25°C mit einer Lösung aus 4,1 g p-Amino-benzoesäure in 150 ml Diethylether und 2,9 g Pyridin versetzt. Man rührte für 2 h bei RT nach. Zur Aufarbeitung wurde das Reaktionsgemisch mit eiskalter 1 n HCl und gesättigter NaCl-Lösung extrahiert. Nach Abtrennung der wäßrigen Phase und Entfernung des Lösungsmittels unter vermindertem Druck wurden 11,7 g (93 % d. Th.) des oben genannten Produkts erhalten, das nach Umkristallisieren aus Isopropanol/Ethanol einen Festpunkt Fp von 187°C aufwies.

Beispiel 7

2-[9(Z)-Retinoyloxy]-3',4'-dimethoxy-acetophenon

Ein Gemisch aus 50 ml Dimethylformamid, 0,75 g Kaliumcarbonat, 2,2 g 2-Chlor-3', 4'-dimethoxy-acetophenon und 3 g 9(Z)-Retinsäure wurde unter Stickstoff und Lichtausschluß 24 h bei RT gerührt. Zur Aufarbeitung wurde das Reaktionsgemisch mit 100 ml Wasser versetzt und 2 h bei RT nachgerührt. Die ausgefallenen Kristalle wurden abgesaugt und nach Umkristallisation aus Isopropanol unter Stickstoff getrocknet. Man erhielt so 3,6 g (96 % d. Th.) kristallines Wertprodukt vom Fp= 130°C.

Beispiel 8

N-[1-(D-Glucopyranosyluronosyl)]-9(Z)-retinsäureamid

Ein Gemisch aus 10 g 9(Z)-Retinsäure, 2,9 g Pyridin in 500 ml Diethylether wurde unter Lichtausschluß unter $N_2$ auf 0°C abgekühlt. Anschließend wurden bei 0°C 3,5 g Thionylchlorid zugetropft und 2 h bei 0°C nachgerührt. Ausgefallenes Pyridiniumhydrochlorid wurde abgetrennt. Das Filtrat wurde danach bei -25°C mit einer Lösung aus 10 g Methyl-(2,3,4-tri-0-acetyl-β-D-glucopyranosyluronosyl)-amin in 150 ml Diethylether und 4,2 g Kaliumcarbonat versetzt. Man rührte für 2 h bei -25°C nach und erwärmte innerhalb von 1 h auf 0°C. Anschließend wurde das Reaktionsgemisch mit 2 g Kaliumcarbonat und 100 ml Methanol versetzt. Zur Entfernung der Schutzgruppen erfolgte nun ein Zusatz einer 20%igen KOH-Lösung in 150 ml Methanol. Das Reaktionsgemisch wurde für 0,5 h auf 60°C erhitzt, danach auf RT abgekühlt. Nach Extraktion mit Heptan/Essigsäureethylester wurden die Wasserphasen mehrfach mit Methylenchlorid extrahiert. Die Methylenchloridphasen wurden danach unter vermindertem Druck eingeengt. Der verbleibende Rückstand wurde durch Chromatographie an Kieselgel in ein 1 : 1-Isomerengemisch der beiden Anomeren aufgetrennt. Man erhält so ein 1 : 1-Isomerengemisch des oben genannten Produkts; in Mengen von 8,2 g (entsprechend 60 % d. Th.) von einem Festpunkt Fp. von 56-58°C.

Beispiel 9

9(Z)-Retinsäure-tocopherylester

6 g 9(Z)-Retinsäure wurden in 150 ml Diisopropylether gelöst und die Lösung auf 5°C abgekühlt. Anschließend wurden 4,6 g Trifluoressigsäureanhydrid zugetropft und das Reaktionsgemisch auf RT erwärmt. Danach wurde die Lösung von 9,6 g D,L-Tocopherol in 10 ml Diisopropylether zugetropft 1 h bei RT nachgerührt. Zur Aufarbeitung wurden dem Ansatz bei 20 bis 25°C 20 ml Ammoniak-Wasser zugesetzt. Nach Phasentrennung wurde die organische Phase mit Wasser gewaschen, getrocknet und danach das Lösungsmittel destillativ entfernt. Man erhielt 12 g (entsprechend 84 % d.Th.) des oben genannten Produktes in Form eines orange-gelben Öls, dessen NMR-Daten mit der formulierten Struktur übereinstimmen.

Beispiel 10

9(Z)-Retinsäure-ergocalciferylester

Ein Gemisch aus 10 g 9(Z)-Retinsäure, 2,9 g Pyridin und 500 ml Diethylether wurde unter Lichtausschluß und $N_2$-Schutzgas auf 0°C abgekühlt, bei 0°C 3,5 g Thionylchlorid zugetropft und das Reaktionsgemisch 2 h bei 0°C nachgerührt. Ausgefallenes Pyridiniumhydrochlorid wurde abgetrennt. Das Filtrat wurde bei -25°C mit einer Lösung von 13,2 g Ergocalciferol in 150 ml Diethylether und mit 4,2 g Kaliumcarbonat versetzt. Man rührte 2 h bei -25°C nach und erwärmte dann innerhalb von 1h auf 0°C. Anschließend wurde das Reaktionsgemisch mit 10%iger wässriger $KHCO_3$-lösung versetzt und dann mit Wasser gewaschen. Nach destillativer Entfernung des Lösungsmittels erhielt man 15 g des oben genannten Produkts in Form eines dunkelorangen Öls. Die NMR-Spektren entsprechen der formulierten Struktur.

## Patentansprüche

1. Ester und Amide der 9(Z)-Retinsäure für die Verwendung zur Behandlung von Carcinomen mit der allgemeinen Formel I

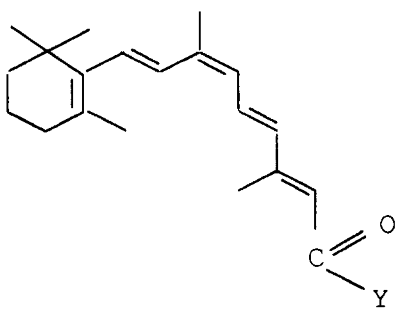 (I) ,

in der Y für folgende Reste stehen kann:

$$-O-CR^1_2-CO-R^2,$$

worin $R^1$
für H oder $C_1$-bis $C_{20}$-Alkyl oder $C_1$-bis $C_{20}$-Alkanoyl und
$R^2$ für $C_1$-bis $C_4$-Alkyl, $-CH_2OH$, $-NR^3_2$ , $-CH_2$-O-CO-$R^3$,

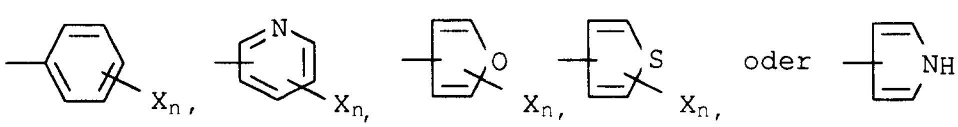

steht, und worin $R^3$ für H oder $C_1$-bis $C_4$-Alkyl steht, X für H, F, Cl, Br, J, -OH, $-OR^3$, $-NH_2$, $-NO_2$, -CN, $-NHR^3$, $-NR^3_2$ , -SH, $SR^3$, -O-CO-$R^3$, -CO-$R^3$, -CHO oder -CO-$OR^3$ steht und n für eine ganze Zahl von 0 bis 5 steht
oder aber Y für

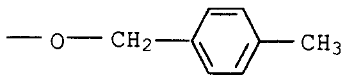 ; 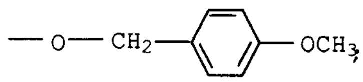

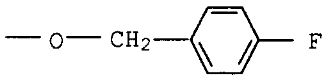 ; 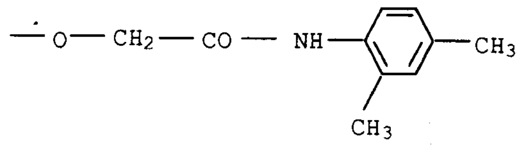 ;

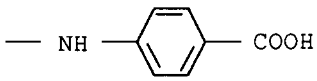 ; 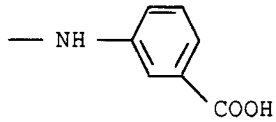 ;

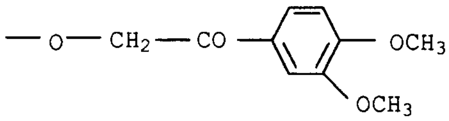

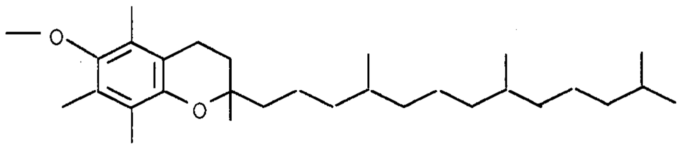 ;

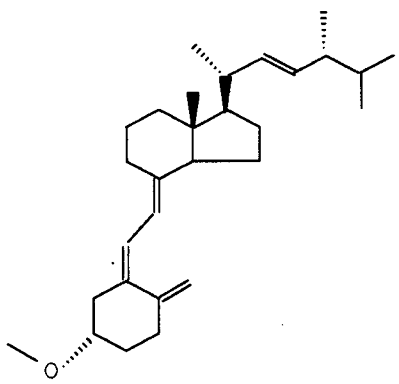

$-O-CHR^4_2$ ; $-O-CHR^3-O-CO-OR^3$

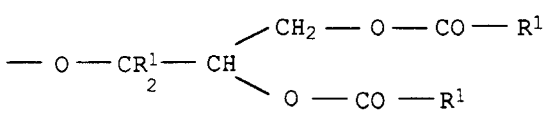

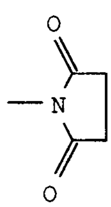 ; 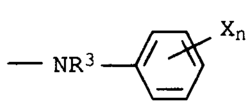 ; 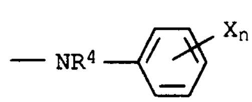 ,

worin $R^1$, $R^3$, X und n die oben angegebene Bedeutung haben und $R^4$ für -CO-$OR^3$, -CO-$R^3$ oder

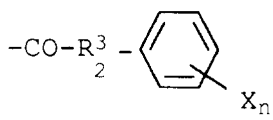

steht,
wobei in den Verbindungen, in denen mehrere Reste $R^1$, $R^2$, $R^3$ und/oder $R^4$ enthalten sind, diese gleich oder verschieden sein können, oder aber
Y für einen der Reste

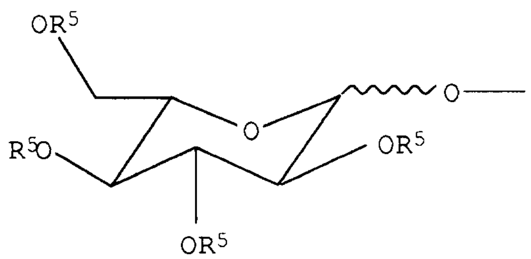

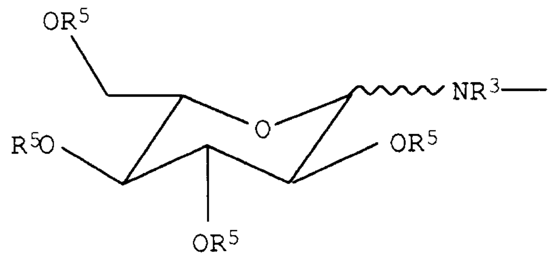

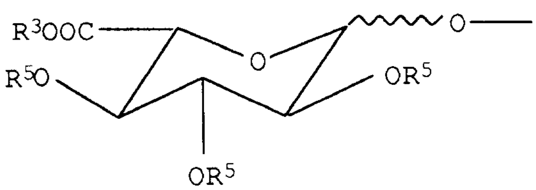

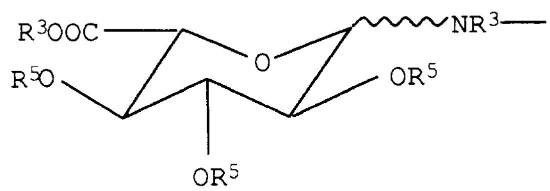

steht,
in der $R^3$ die oben angegebene Bedeutung hat und $R^5$ für H oder einen $C_1$-bis $C_4$-Alkylrest oder $C_1$ -bis $C_4$-Alkanoylrest steht.

**2.** Ester und Amide der 9(Z)-Retinsäure für die Verwendung bei der Behandlung von Carcinomen mit der allgemeinen Formel I gemäß Anspruch 1, worin Y für den Rest

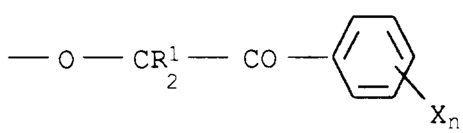

steht, in der $R^1$ für H,
X für F, -OH, $-OCH_3$, $-NH_2$, -CN, $-NHCH_3$ oder $-N(CH_3)_2$ steht und n eine ganze Zahl von 1 bis 3 bedeutet.

**3.** Amide der 9(Z)-Retinsäure für die Verwendung bei der Behandlung von Carcinomen mit der allgemeinen Formel I gemäß Anspruch 1,
worin Y für einen der Reste

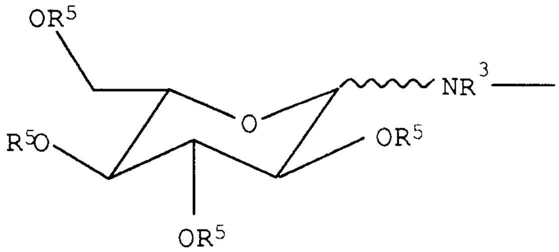

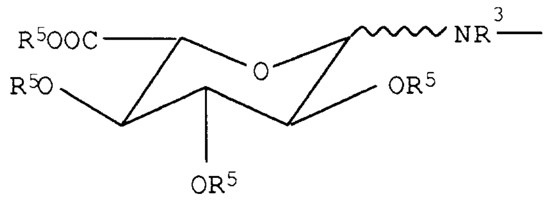

worin $R^5$ für H, $C_1$-bis $C_4$-Alkyl oder $C_1$-bis $C_4$-Alkanoyl und $R^3$ für H, $C_1$-bis $C_4$-Alkanoyl oder $C_1$-bis $C_4$-Alkyl stehen.

**4.** Ester und Amide der 9(Z)-Retinsäure der allgemeinen Formel I

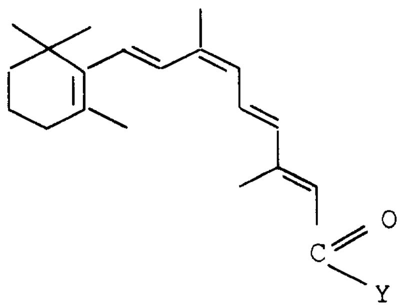

(I) ,

in der Y für

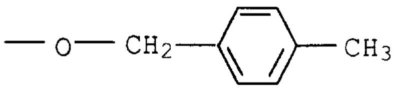 ; 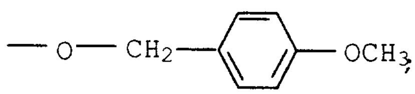 ;

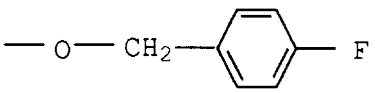 ; 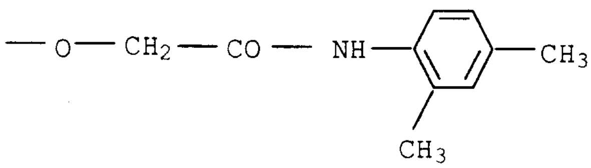 ;

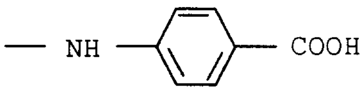 ; 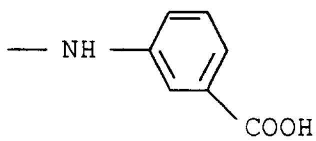 ;

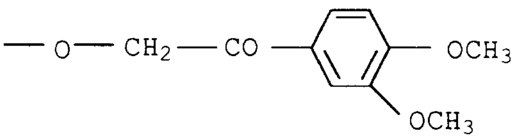

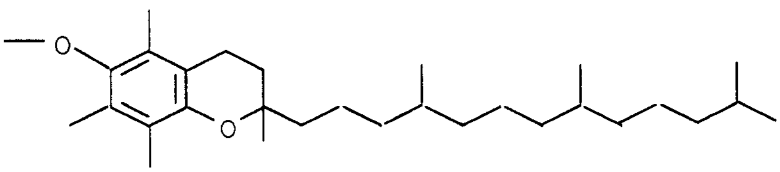 ;

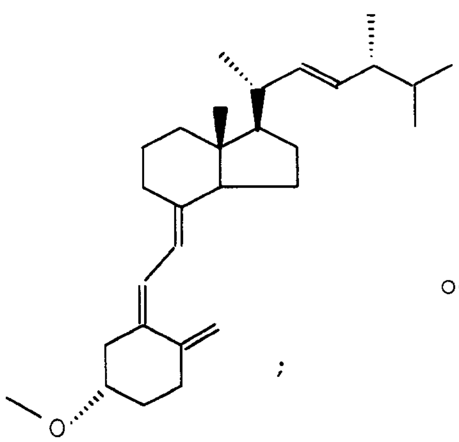 ;

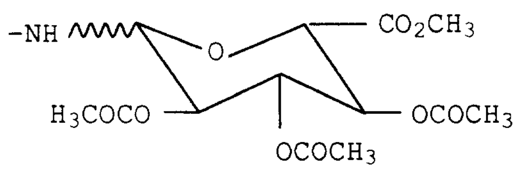 ;

oder 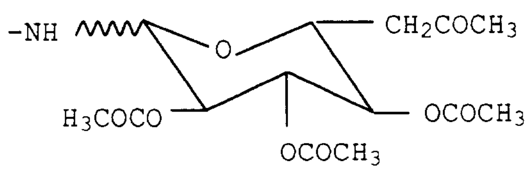

steht.

5. Pharmazeutische Zusammensetzung enthaltend Ester oder Amide der 9(Z)-Retinsäure gemäß den Ansprüchen 1 bis 4 neben üblichen pharmazeutischen Trägerstoffen oder Verdünnungsmitteln und üblichen pharmazeutisch-technischen Hilfsstoffen.

6. Verwendung von Estern oder Amiden der 9(Z)-Retinsäure gemäß einem der Ansprüche 1 bis 4 in Form von Pasten, Gelen, Salben, Cremes, Lotionen, Puder, Lösungen oder Emulsionen enthaltend 0,001 bis 5 % dieser Verbindung für die lokale Behandlung von Carcinomen oder Praecancerosen.

7. Verwendung von Estern oder Amiden der 9(Z)-Retinsäure gemäß einem der Ansprüche 1 bis 4 in Form von Tabletten, Filmtabletten, Dragees, Kapseln, Pillen, Pulver, Granulat, Lösungen oder Suspensionen für die systemische Behandlung von Carcinomen oder Praecancerosen.

8. Verwendung von Pharmazeutischen Zusammensetzungen gemäß Anspruch 5 für die lokale oder systemische Prophylaxe von Carcinomen.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 96 10 9672

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| X | CHEMICAL ABSTRACTS, vol. 118, no. 5, 1.Februar 1993 Columbus, Ohio, US; abstract no. 039206, TOYODA H ET AL: "Preparation vitamin A acid ester compounds as antitumor agents and ulcer therapeutics for skin and digestive tract" XP002015027 * Zusammenfassung * & JP-A-04 244 058 (NISSHIN FLOUR MILLING CO., LTD.) 1.September 1992<br>---<br>-/-- | 1,4 | C07C403/20<br>C07C403/18<br>C07D311/72<br>C07C401/00<br>C07H13/04<br>A61K31/20<br>A61K31/07<br>A61K31/595<br>A61K31/355<br>A61K31/70 |

RECHERCHIERTE SACHGEBIETE (Int.Cl.6)

C07C
C07D
C07H
A61K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 3.Oktober 1996 | Bonnevalle, E |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 96 10 9672

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | CHEMICAL ABSTRACTS, vol. 118, no. 5, 1.Februar 1993 Columbus, Ohio, US; abstract no. 039207, TOYODA H ET AL: "Preparation of vitamin A acid ester compounds as antitumor agents and ulcer therapeutics for skin and digestive tract" XP002015028 * Zusammenfassung * & JP-A-04 244 076 (NISSHIN FLOUR MILLING CO., LTD.) 1.September 1992 --- | 1,4 | |
| X | EP-A-0 552 624 (HOFFMANN LA ROCHE) * Seite 3, Zeile 36 - Zeile 39; Ansprüche * --- | 1,4 | RECHERCHIERTE SACHGEBIETE (Int.Cl.6) |
| Y | CHEMICAL ABSTRACTS, vol. 120, no. 19, 9.Mai 1994 Columbus, Ohio, US; abstract no. 235378, SASS J O ET AL: "9-Cis-retinoyl-.beta.-D-glucuronide is a major metabolite of 9-cis-retinoic acid. [Erratum to document cited in CA120(13):152923n]" XP002015029 * Zusammenfassung * & LIFE SCI. (LIFSAK,00243205);94; VOL.54 (17); PP.PL 311, FREIE UNIV. BERLIN;INST. TOXIKOL. EMBRYOPHARMAKOL.; BERLIN; D-14195; GERMANY (DE), --- | 1 | |
| | -/-- | | |

EPO FORM 1503 03.82 (P04C12)

Europäisches Patentamt

# EUROPÄISCHER TEILRECHERCHENBERICHT

Nummer der Anmeldung
EP 96 10 9672

| EINSCHLÄGIGE DOKUMENTE | | | |
|---|---|---|---|
| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
| Y | CHEMICAL ABSTRACTS, vol. 122, no. 11, 13.März 1995 Columbus, Ohio, US; abstract no. 129040, PATHIRANA C ET AL: "Identification of an activator of the retinoid X receptor" XP002015030 * Zusammenfassung * & J. NAT. PROD. (JNPRDF,01633864);94; VOL.57 (10); PP.1458-61, LIGAND PHARM. INC.;SAN DIEGO; 92121; CA; USA (US), --- | 1 | |
| Y | ARZNEIMITTEL FORSCHUNG DRUG RESEARCH., Bd. 43, Nr. 4, 1993, AULENDORF DE, Seiten 487-490, XP002015026 F. KALEAGASIOGLU ET AL.: "Antiproliferative activity of retinoic acid and some novel retinoid derivatives in breast and colorectal cancer cell lines of human origin" * das ganze Dokument * --- | 1 | RECHERCHIERTE SACHGEBIETE (Int.Cl.6) |
| A | FR-A-2 212 135 (BASF AG) 26.Juli 1974 * Ansprüche * --- | 1,4 | |
| A | US-A-4 677 120 (PARISH HARLIE A ET AL) * Ansprüche * ----- | 1,4 | |

EPO FORM 1503 03.82 (P04C12)

Europäisches Patentamt

EP 96 10 9672 - C -

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:
Unvollständig recherchierte Patentansprüche: 1-8
Nicht recherchierte Patentansprüche:

Grund für die Beschränkung der Recherche: Die Zahl, der in den Patentansprüche 1 bis 8 genannten Verbindungen, ist so gross das eine vollständige Recherche nicht möglich ist. Die Recherche beschränkt sich deshalb ausschliesslich auf die Verbindungen beschrieben in den Beispiele 1 bis 10.